# EUROPEAN PATENT APPLICATION

(11) **EP 4 064 295 A1**
(43) Date of publication of application: **28.09.2022**
(21) Application number: 21164522.1
(22) Date of filing: 24.03.2021
(51) Int. Cl.: G16H 50/50, G16H 50/70

(54) **RESPIRATORY MUSCLE CAPABILITY**

(71) Applicant: Koninklijke Philips N.V., 5656 AG Eindhoven (NL)
(72) Inventor: SABCZYNSKI, Jörg, 5656 AE Eindhoven (NL); WIEMKER, Rafael, 5656 AE Eindhoven (NL); HENDRIKS, Cornelis Petrus, 5656 AE Eindhoven (NL)
(74) Representative: Philips Intellectual Property & Standards

(57) **Abstract**

A method for indicating the capability of a subject's respiratory muscles is provided. The method comprises adapting a shape model of the respiratory system to medical images of a subject; obtaining a biomechanical model of the subject's thorax; predicting respiratory volume change of the subject using the aforementioned models and then generating an indication of respiratory muscle capability based on the prediction results. The method is used for COPD diagnosis and treatment selection.

## Description

### FIELD OF THE INVENTION

This present invention relates to the field of medical modelling, and more particularly to indicating a capability of respiratory muscles of a subject.

### BACKGROUND OF THE INVENTION

Chronic obstructive pulmonary disease (COPD) has a significant impact on the capability (i.e. effectiveness) of respiratory muscles, eventually leading to exercise intolerance, a factor that can significantly affect quality of life. COPD subjects suffer from structural alterations of the respiratory muscles and skeletal system, which result in changes to the respiratory biomechanics. Examples of these structural alterations, which are caused by lung hyperinflation, include a decreased curvature of the diaphragm; a worsening of the length-tension relationship of the diaphragm; and an increase in the elastic recoil of the ribcage. Thus, the geometry of the diaphragm and the ribcage has a strong influence on the maximal transdiaphragmatic pressure the subject can generate.

Additionally, in severe cases of hyperinflation, the diaphragm muscle fibers can develop an abnormal orientation that worsens the symptoms of COPD. Therefore, pulmonologists are interested to know the mass and/or the capability of the respiratory muscles of a subject, or the work of breathing, such that respiratory diseases can be diagnosed and appropriate treatment can be selected accordingly.

Professionals currently determine if the work of breathing is increased by gestalt or by examining the subject looking for signs of increased breathing effort such as nasal flaring, the contraction of sternomastoid and thoracoabdominal paradox. However, the clinal need currently exists to determine the capability of the respiratory muscles in a noninvasive, safe, easy and objective/quantifiable manner.

### SUMMARY OF THE INVENTION

The invention is defined by the claims.

According to examples in accordance with an aspect of the invention, there is provided a method for indicating a capability of respiratory muscles of a subject comprising:
adapting a shape model of the respiratory system to a medical image of the subject's respiratory system to obtain a subject-specific shape model;
obtaining a biomechanical model of the subject's thorax;
predicting respiratory volume change of the subject due to respiratory muscle contraction based on the subject-specific shape model and the biomechanical model; and
generating an indication of respiratory muscle capability based on the prediction results.

Proposed embodiments may enable users to obtain an indication of a subject's respiratory muscle capability by adapting a shape model (such as a statistical shape model for example) to a medical image of the subject and obtaining a biomechanical model of the subject's thorax. The respiratory volume change of the subject, caused by contraction of the subject's respiratory muscles, may be predicted using the shape model and biomechanical model. From this prediction, an indication of the subject's respiratory muscle capability may be generated. The method may be used during chronic obstructive pulmonary disease (COPD) diagnosis, treatment selection, therapy response monitoring etc. Improved (e.g. more accurate) clinical diagnosis may therefore be facilitated by proposed embodiments.

In particular, embodiments may be used in relation to a subject (e.g. a patient) so as to assist and/or optimize medical assessment, therapy and/or treatment for the subject. Such embodiments may support improved clinial assessment and/or planning. Improved Clinical Decision Support (CDS) may therefore be provided by proposed concepts.

Proposed embodiments that quantify the influence of the diaphragm and ribcage geometry on the work that is needed for breathing and the capability of the respiratory muscles may help various clinical needs.

Respiratory diseases such as COPD can cause structural alterations of the respiratory muscles and skeletal system, particularly to the diaphragm and ribcage. Proposed embodiments may employ a shape model of the respiratory system that can be adapted to medical images of a subject, producing a subject-specific shape model, wherein the shape model may preferably be a statistical shape model. The subject-specific shape model may then be compared to the shape models of subjects previously diagnosed with different respiratory diseases, such that similarities between models may be identified. Furthermore, a library of shape models associated with different respiratory diseases may be generated, such that the future identification of structural alterations and the symptoms of other respiratory diseases may be simplified, enabling assisted diagnosis.

Additionally, according to proposed embodiments, a biomechanical model of the subject's thorax may be obtained. The biomechanical model undertakes muscle contraction and muscle activation pattern simulations, such that the motion of the thorax may be predicted, and in turn the respiratory volume change of the subject may also be predicted. From these results, an indication of the subject's respiratory muscle capability may be generated, and this information may help for early diagnosis of respiratory diseases.

Subjects with pre-existing respiratory diseases may benefit from an increased quantity and quality of information gathered regarding their respiratory system when using proposed embodiments over conventional methods. For example, the capability of the respiratory muscles and the work of breathing are very useful to pulmonologists. Consequently, proposed embodiments may enable improved treatment selection. As an example, the consideration of exercises to strengthen muscles may be deemed suitable or not. Furthermore, repeated use of proposed embodiments may allow for treatment response monitoring, such that the most suitable treatment can be selected for subjects depending on their response to previous treatments. This process may be much more effective than when using conventional methods.

In some embodiments, the shape model may comprise models of the ribcage (including vertebrae and sternum); the respiratory muscles (including diaphragm with central tendon and left and right crus); the muscle fibers emerging from the xiphoid process, ribs, vertebra and left and right crus; the intercostal muscles and the costal cartilages. The detailed nature of the shape model may enable more accurate predictions of respiratory volume changes of the subject, which in turn allows the generation of more accurate indications of respiratory muscle capability.

Other embodiments may adapt a shape model of the respiratory system to the subject's medical image using a method of segmentation, wherein the medical image is segmented into a plurality of image segments. The shape model may then be adapted to each of the image segments. The segmentation of the medical image may support a more reliable adaption of the shape model, such that the shape model may provide a more accurate representation of the subject. The segmentation process may also be implemented using a machine learning algorithm to improve efficiency.

Some embodiments may obtain the biomechanical model of the subject from the subject-specific shape model. Bony structures in the shape model may be modelled as rigid in the biomechanical model. Additionally, a pair of bony structures in the shape model may be connected with one another in the biomechanical model via a joint that forms constraint for the motion of bones. Muscular structures in the shape model may be modelled as flexible in the biomechanical model. Whilst some muscles such as the tendinous parts of the diaphragm remain at a constant length during breathing, other muscles contract and elongate. Hence muscle contraction is modelled by actively shortening the muscles in the biomechanical model. This in turn drives the motion of the tendons and bones. The simulation of respiratory motion is made as lifelike as possible to ensure accurate indications of the respiratory muscle capability may be generated.

Further, in some embodiments, the prediction of respiratory volume change may comprise the following process. Initially, muscle activation patterns of the subject based on the subject-specific shape model and biomechanical model may be simulated. The thorax motion of the subject may then be predicted based on the simulation results. Finally, based on the predicted thorax motion, the respiratory volume change may then be predicted. This process may provide a more realistic prediction of respiratory volume change.

To add to the previous embodiments, other embodiments may predict the motion of the subject's thorax by first simulating the resulting muscle contractions for a plurality of muscle activation patterns and activation strengths, then simulating the change in thorax geometry for each muscle contraction pattern. This may provide the benefit of a more accurate prediction of thorax motion.

In other embodiments, simulations of changes in thorax geometry may involve the following process. To start, the anatomical cross-sectional area (ACSA) of the respiratory muscles from the medical image of the subject's respiratory system may be estimated. The direction of the muscle fibers based on the subject-specific shape model may then be approximated. The physiological cross-sectional area (PCSA) of respiratory muscles may then be calculated. Such a process may enable more subject-specific thorax geometry simulations.

In some embodiments, the prediction of respiratory volume change may involve the following estimations or analysis. If two or more medical images of the patient from breathing states are available, the images may be analyzed to identify a single or multiple muscle activation patterns. In addition, if there are parameters required to predict thorax motion that are unknown, the values may be estimated based on parameters available from population studies. Alternatively, unknown parameter values may be estimated by creating a subject-specific computational fluid dynamics (CFD) based flow simulation. In this way, respiratory volume change predictions which may have been previously difficult to make are simplified and made possible.

Embodiments may generate an indication of respiratory muscle capability using different variables. The possible combinations of variables include the quotient of volume change and muscle activation strength, the quotient of volume change and relative muscle contraction length, and the pressure change induced by a muscle activation pattern in equilibrium. Depending on the subject, the most appropriate method to generate an indication of respiratory muscle capability may be chosen, thereby making the indication more versatile.

Other embodiments may use computed tomography (CT) or magnetic resonance imaging (MRI) scans as medical images which may provide the benefit of more accurate shape modelling.

Embodiments may obtain a biomechanical model that comprises a model created by finite element method (FEM). Such a model may enable more reliable muscle contraction simulations.

Embodiments may comprise a computer program product comprising computer program code means which, when executed on a computing device having a processing system, cause the processing system to perform a method for indicating a capability of respiratory muscle of a subject. The method comprises: adapting a shape model of the respiratory system to a medical image of the subject's respiratory system to obtain a subject-specific shape model; obtaining a biomechanical model of the subject's thorax; predicting respiratory volume change of the subject due to respiratory muscle contraction based on the subject-specific shape model and the biomechanical model; and generating an indication of respiratory muscle capability based on the prediction results.

Some embodiments may comprise a system for indicating a capability of respiratory muscles of a subject, wherein the system may comprise an adaption unit, an interface unit, a prediction unit and a generational unit. The adaption unit may be configured to adapt a shape model of the respiratory system to a medical image of the subject's respiratory system to obtain a subject-specific shape model. The interface unit may be configured to obtain a biomechanical model of the subject's thorax. The prediction unit may be configured to predict respiratory volume change of the subject due to muscle contraction based on the subject-specific shape model and biomechanical model. The generational unit may be configured to generate an indication of respiratory muscle capability based on prediction results.

In other embodiments, the adaption unit may comprise a segmentation component and an adaptation component. The segmentation component may be configured to segment the medical image into a plurality of image segments. The adaptation component may be configured to adapt the shape model of the respiratory system to each of the image segments.

In further embodiments, the prediction unit may be configured to perform the following functions. The prediction unit may simulate single or multiple muscle activation patterns of the subject based on the subject-specific shape model and biomechanical model. The prediction unit may also predict thorax motion of the subject based on the simulation results, and the unit may calculate a respiratory volume change based on the predicted thorax motion.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows a method for generating an indication of respiratory muscle capability from an input of a medical image of a subject's respiratory system;
Figure 2 shows a shape model of the respiratory system with biomechanical components;
Figure 3 shows a method for predicting respiratory volume change of a subject due to respiratory muscle contraction;
Figure 4 shows a system for generating an indication of respiratory muscle capability from an input of a medical image of a subject's respiratory system;
Figure 5 shows the application of a biophysical model to inspiratory/expiratory medical images; and
Figure 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage. It should be understood that the Figures are merely schematic and are not drawn to scale. It should also be understood that the same reference numerals are used throughout the Figures to indicate the same or similar parts.

Propose concepts provide for generation of an indication of the capability of the respiratory muscles of a subject. Embodiments may therefore be used for chronic obstructive pulmonary disease (COPD) diagnosis, treatment selection and progress monitoring. Embodiments utilize a shape model that is personalized to the subject by adapting the model to medical images of the subject, wherein the shape model is preferably a statistical shape model. Examples of medical images include computed tomography (CT) or magnetic resonance imaging (MRI) scans. A biomechanical model of the subject's thorax is also obtained. The respiratory volume change of the subject due to respiratory muscle contraction is predicted using both models, and an indication of the subject's respiratory muscle capability is determined using quotients derived from the results of the respiratory volume change prediction.

Implementations in accordance with the present disclosure relate to various systems, adaptions, and/or methods of quantifying respiratory muscle capability pertaining to diagnosing and treating respiratory diseases such as COPD. According to proposed concepts, a number of possible solutions may be implemented separately or jointly. That is, although these possible solutions may be described below separately, two or more of these possible solutions may be implemented in one combination or another.

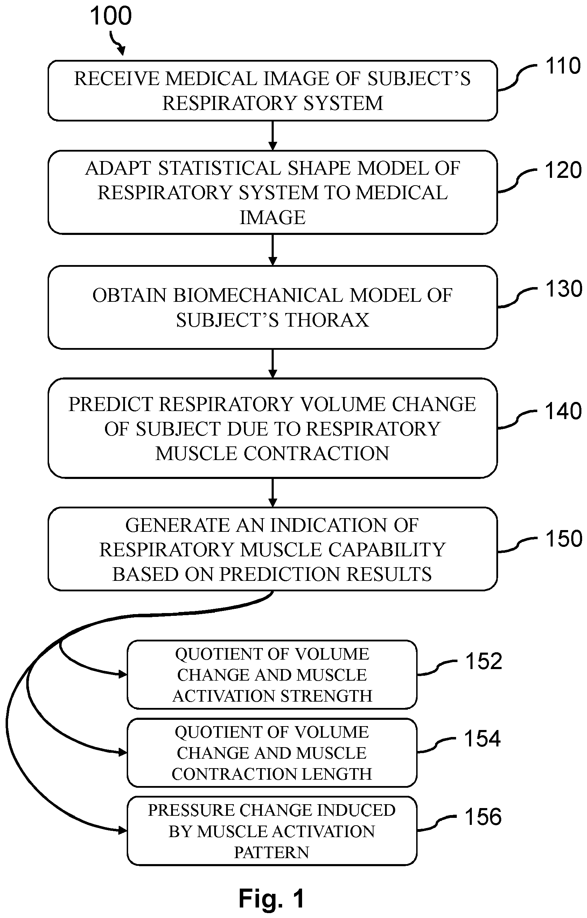

Figure 1 shows a method for generating an indication of respiratory muscle capability from an input of a medical image of a subject's respiratory system 100 according to a proposed embodiment.

In step 110, a medical image of the subject's respiratory system is received. The medical image comprises a computed tomography (CT) scan or a magnetic resonance imaging (MRI) scan. The CT scan can be either a diagnostic or a screening low-dose CT images. Multiple medical images may be received, with useful information contained within images of the subject's respiratory system at rest, or during inspiration/expiration. CT and MRI scans are used as medical images as contain sufficient information to enable a detailed and accurate adaption of the shape model to the subject's respiratory system.

In step 120, a shape model of the respiratory system is adapted to the subject's medical image to obtain a subject-specific shape model of the respiratory system, wherein the shape model is preferably a statistical shape model. In order for the shape model to be of ample detail, the model must comprise at least one of: a model of the ribcage including vertebrae and sternum; a model of the respiratory muscles including the diaphragm with central tendon and left and right crus; a model of the muscle fibers emerging from the xiphoid process, ribs, vertebra and left and right crus; and a model of the intercostal muscles and costal cartilages. The shape model comprises all structures that contribute to breathing. To enable biomechanical modelling, the model must contain the muscles driving inspiration and expiration i.e. specifically the diaphragm and intercostal muscles, hence it is of particular importance that the diaphragm and muscle fibers are modelled correctly.

To aid the adaption of the shape model to the medical image of the subject's respiratory system, the medical image can be segmented into a plurality of image segments, with the segments comprising bones, muscles, diaphragm and lungs. The shape model of the respiratory model is adapted to each of the image segments. Segmentation is undertaken by either deep convolutional neural networks (CNNs) or by more traditional approaches, and is particularly useful when multiple medical images from inspiratory/expiratory states are received. Additionally, segmentation can be implemented by a machine learning algorithm to improve efficiency.

In step 130, a biomechanical model of the subject's thorax is obtained. Possible methods for obtaining the biomechanical model comprise generation by machine learning algorithm and manual annotation of all required biomechanical elements. The biomechanical model may comprise a model created by finite element method, FEM. The biomechanical model may also comprise other model types, such as one generated using deep convolutional neural networks.

The biomechanical model may be obtained from the shape model, in which case the biomechanical model is obtained by: modelling a bony structure in the subject-specific shape model as rigid; connecting a pair of bony structures in the subject-specific shape model with one another via a joint; and modelling a muscular structure in the subject-specific shape model as flexible. In the biomechanical model, joints form constraints for the motion of bones. Whilst some muscles such as the tendinous parts of the diaphragm remain at a constant length during breathing, other muscles contract and elongate. Hence muscle contraction is modelled by actively shortening the muscles in the biomechanical model. This in turn drives the motion of the tendons and bones. The diaphragm shape is constrained by intra-thoracic and intra-abdominal pressures. By adhering to the aforementioned process, the biomechanical model provides realistic simulations of the subject's respiratory motion.

In step 140, the respiratory volume change of the subject due to respiratory muscle contraction is predicted using the biomechanical and subject-specific shape models of steps 120, 130. Predicting respiratory volume change comprises simulating a muscle activation pattern of the subject based on the subject-specific shape model and biomechanical model; then predicting thorax motion of the subject based on the simulation results; before calculating a respiratory volume change based on the predicted thorax motion.

Predicting thorax motion comprises at least one of: simulating muscle contraction patterns for a plurality of muscle activation patterns and activation strengths; and simulating a change in thorax geometry for the muscle contraction patterns.

Further, simulating a change in thorax geometry comprises at least one of: estimating the anatomical cross-sectional area, ACSA, of respiratory muscles from a medical image of the subject's respiratory system; approximating the direction of muscle fibers based on the subject-specific shape model; and calculating the physiological cross-sectional area, PCSA, of respiratory muscles.

Predicting respiratory volume change further comprises at least one of: analyzing one or more medical images from breathing states to identify a single or multiple muscle activation patterns; estimating an unknown parameter value based on parameters available from population studies; and estimating an unknown parameter value by creating a subject-specific computational fluid dynamics, CFD, based flow simulation.

In step 150, an indication of respiratory muscle capability is generated based on the prediction results. The method of generating an indication of respiratory muscle capability is undertaken using one of the steps 152, 154, 156. In step 152, the respiratory muscle capability indication is generated using a quotient of volume change and muscle activation strength. In step 154, the respiratory muscle capability indication is generated using a quotient of volume change and muscle contraction length. In step 156, the respiratory muscle capability indication is generated using the pressure change induced by muscle activation patterns. By having different methods for generating an indication of respiratory muscle capability, the most appropriate method can be determined depending on the subject.

The presented method for generating an indication of respiratory muscle capability is used during COPD diagnosis, treatment selection, planning of COPD-related lung volume reduction surgery, therapy response monitoring etc.

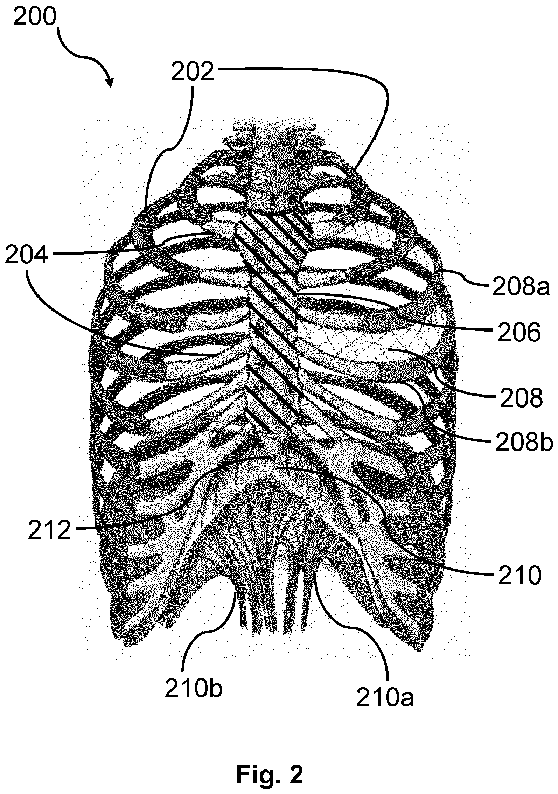

Figure 2 shows a shape model of the respiratory system with biomechanical components 200 according to a proposed embodiments. The model comprises ribs and vertebrae 202, costal cartilages connecting the ribs to the sternum 204, the sternum 206, intercostal muscles 208 and the diaphragm 210. The sternum 206 is distinguished from the costal cartilages 204 and xiphoid process 212 by hatching. The intercostal muscles 208 are only demonstrated between two ribs 208a, 208b, though it should be noted that this is for diagrammatic purposes only and that the shape model will show intercostal muscles between all ribs 202. The diaphragm 210 comprises a left crus 210a and right crus 210b. Further, the shape model will comprise muscle fibers emerging from different structures along the costal margin, such as the xiphoid process 212, ribs and vertebrae 202 and left and right crus 210a, 210b. Hence, the shape model comprises all structures which contribute to breathing and requires all of the components stated previously to provide a model of ample detail. To enable biomechanical modelling, the model must contain the muscles driving inspiration and expiration i.e. specifically the diaphragm 210 and intercostal muscles 208. It is of particular importance that the diaphragm and muscle fibers are modelled correctly to ensure accurate simulations can be carried out.

To aid the adaption of the shape model to the medical image of the subject's respiratory system, the medical image can be segmented into a plurality of image segments, with the segments comprising bones, muscles, diaphragm and lungs. The shape model of the respiratory model is adapted to each of the image segments. Segmentation is undertaken by either deep convolutional neural networks (CNNs) or by more traditional approaches, and is particularly useful when multiple medical images from inspiratory/expiratory states are received. Additionally, segmentation can be implemented by a machine learning algorithm to improve efficiency.

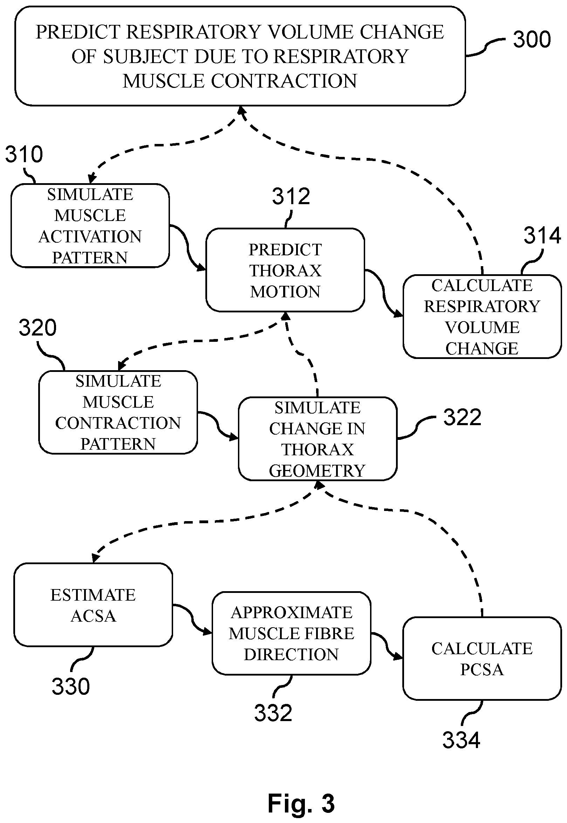

Figure 3 shows step 140 from Figure 1, wherein the respiratory volume change of the subject due to respiratory muscle contraction is predicted using the biomechanical and subject-specific shape models, demonstrated by step 300. Predicting respiratory volume change comprises three steps 310, 312, 314. In step 310, a single or multiple muscle activation patterns of the subject based on the subject-specific shape model and biomechanical model are predicted. In step 312, the subject's thorax motion is predicted based on the results of the simulation 310. In step 314, the respiratory volume change based on the predicted thorax motion 312 is calculated.

Further, predicting thorax motion comprises at least one of two steps 320, 322. In step 320, muscle contraction patterns are simulated for a plurality of muscle activation patterns and activation strengths. If parameters necessary for step 320, e.g. abdominal pressure or bronchial resistance, are unknown, then the values can be estimated based on parameters available from population studies, or can be estimated by creating a subject-specific computational fluid dynamics, CFD, based flow simulation. Further, to support the identification of muscle activation patterns for step 320, two or more medical images from inspiratory/expiratory breathing states may be analyzed. In step 322, a change in thorax geometry is simulated for the muscle contraction pattern simulations 320. Similarly, the identification of muscle activation patterns by analysis of two or more medical images from inspiratory/expiratory breathing states may also be of use for step 322.

Further, simulating a change in thorax geometry comprises at least one of three steps 330, 332, 334. In step 330, the anatomical cross-sectional area, ACSA, of the subject's respiratory muscles is estimated, wherein the area is estimated using a medical image of the subject's respiratory system. In step 332, the directions of muscle fibers are approximated with knowledge or population-based information on the direction and strength of muscle fibers integrated into the subject-specific shape model. In step 334, the physiological cross-sectional area, PCSA, of respiratory muscles is calculated. This process involving steps 330, 332, 334 determines subject-specific contraction patterns, used to improve simulations of changes in thorax geometry 322.

For the planning of lung volume reduction surgery, the biomechanical model can be used to estimate post-operative respiratory muscle capability by decreasing the intra-thoracic volume in simulations 310, 320, 322.

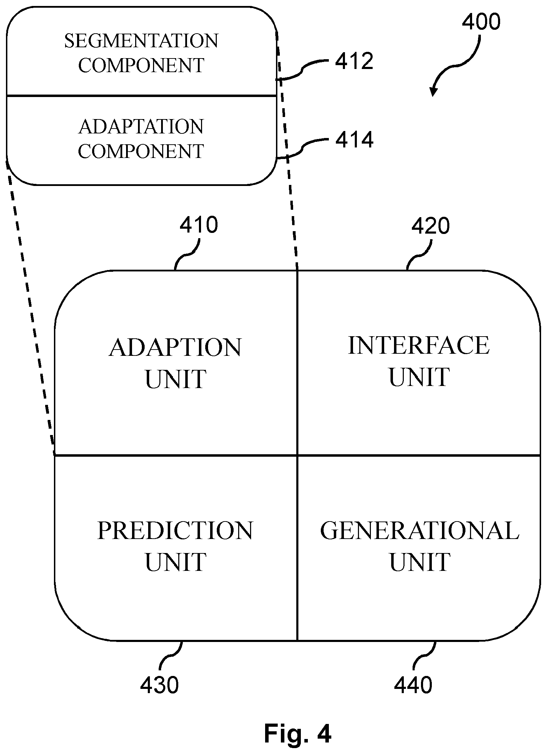

Figure 4 shows a system for indicating a capability of respiratory muscles of a subject 400. The system comprises an adaption unit 410, an interface unit 420, a prediction unit 430 and a generational unit 440.

The adaption unit 410 is configured to adapt a shape model of the respiratory system to a medical image of the subject's respiratory system to obtain a subject-specific shape model. The adaption unit 410 further comprises a segmentation component 412 and an adaptation component 414. The segmentation component 412 is configured to segment the medical image into a plurality of image segments. The adaptation component 414 is configured to adapt the shape model of the respiratory system to each of the image segments.

The interface unit 420 is configured to obtain a biomechanical model of the subject's thorax.

The prediction unit 430 is configured to predict respiratory volume change of the subject due to muscle contraction based on the subject-specific shape model and biomechanical model. The prediction unit 430 is further configured to: simulate a muscle activation pattern of the subject 400 based on the subject-specific shape model and biomechanical model; predict thorax motion of the subject based on the simulation results; and calculate a respiratory volume change based on the predicted thorax motion.

The generational unit 440 is configured to generate an indication of respiratory muscle capability based on prediction results.

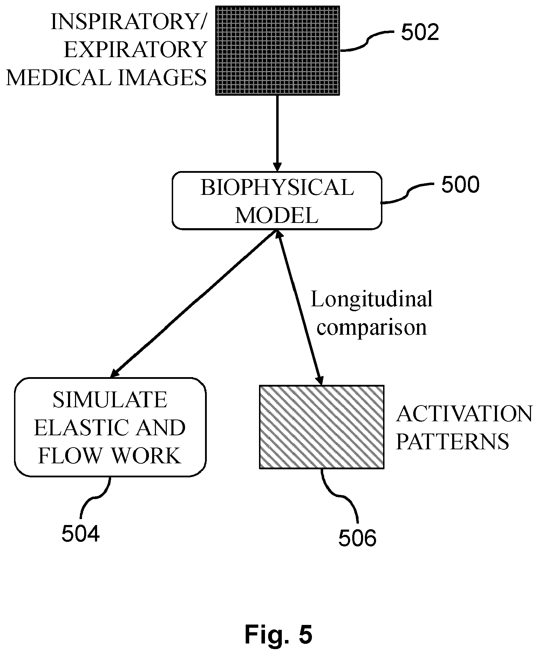

Figure 5 shows the application of a biophysical model 500 to an inspiratory/expiratory medical image 502. The biophysical model 500 comprises tissue deformation and air flow, from which the elastic and flow work can be simulated 504 to find total work. The quotient of total work and muscle mass provides additional information. Alternatively, the biophysical model and activation patterns 506 can be compared longitudinally for a subject as a means for monitoring progression and therapy response.

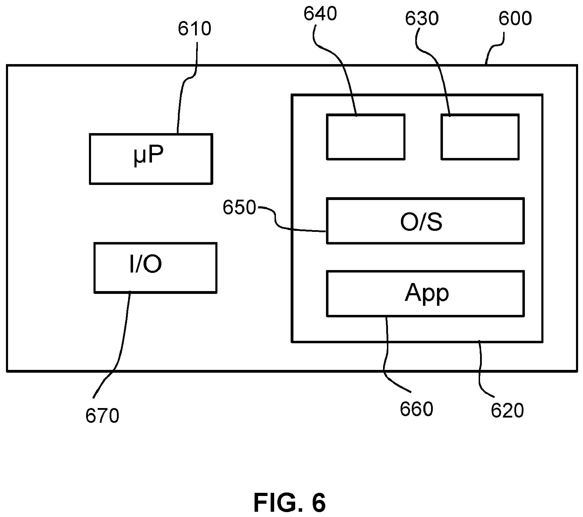

By way of further example, Figure 6 illustrates an example of a computer 600 within which one or more parts of an embodiment may be employed. Various operations discussed above may utilize the capabilities of the computer 600. For example, for indicating a capability of respiratory muscles of a subject may be incorporated in any element, module, application, and/or component discussed herein. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620, and one or more I/O devices 670 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 410.

The software in the memory 620 may include one or more separate programs, each of which comprises an ordered listing of executable instructions for implementing logical functions. The software in the memory 620 includes a suitable operating system (O/S) 650, compiler 640, source code 630, and one or more applications 660 in accordance with exemplary embodiments. As illustrated, the application 660 comprises numerous functional components for implementing the features and operations of the exemplary embodiments. The application 660 of the computer 600 may represent various applications, computational units, logic, functional units, processes, operations, virtual entities, and/or modules in accordance with exemplary embodiments, but the application 660 is not meant to be a limitation.

The operating system 650 controls the execution of other computer programs, and provides scheduling, input-output control, file and data management, memory management, and communication control and related services. It is contemplated by the inventors that the application 460 for implementing exemplary embodiments may be applicable on all commercially available operating systems.

Application 660 may be a source program, executable program (object code), script, or any other entity comprising a set of instructions to be performed. When a source program, then the program is usually translated via a compiler (such as the compiler 640), assembler, interpreter, or the like, which may or may not be included within the memory 620, so as to operate properly in connection with the O/S 650. Furthermore, the application 460 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 670 may include input devices such as, for example but not limited to, a mouse, keyboard, scanner, microphone, camera, etc. Furthermore, the I/O devices 670 may also include output devices, for example but not limited to a printer, display, etc. Finally, the I/O devices 670 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 670 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at startup, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 660 and the O/S 650 are read, in whole or in part, by the processor 610, perhaps buffered within the processor 610, and then executed.

When the application 660 is implemented in software it should be noted that the application 660 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 660 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfill the functions of several items recited in the claims.

It will be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, the system makes use of a processor to perform the data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

## Claims

1. A method for indicating a capability of respiratory muscles of a subject comprising:
adapting (120) a shape model of the respiratory system to a medical image of the subject's respiratory system to obtain a subject-specific shape model;
obtaining (130) a biomechanical model of the subject's thorax;
predicting (140) respiratory volume change of the subject due to respiratory muscle contraction based on the subject-specific shape model and the biomechanical model; and
generating (150) an indication of respiratory muscle capability based on the prediction results.

2. The method according to claim 1, wherein the shape model comprises at least one of:
a model of the ribcage including vertebrae and sternum;
a model of the respiratory muscles including the diaphragm with central tendon and left and right crus;
a model of the muscle fibers emerging from the xiphoid process, ribs, vertebra and left and right crus; and
a model of the intercostal muscles and costal cartilages.

3. The method according to claim 1 or 2, wherein adapting (120) the shape model of the respiratory system comprises:
segmenting the medical image into a plurality of image segments; and
adapting the shape model of the respiratory system to each of the image segments.

4. The method according to any of claims 1 to 3, wherein obtaining (130) the biomechanical model of the thorax comprises at least one of:
modelling a bony structure in the subject-specific shape model as rigid;
connecting a pair of bony structures in the subject-specific shape model with one another via a joint; and
modelling a muscular structure in the subject-specific shape model as flexible.

5. The method of any of claims 1 to 4, wherein predicting (140) respiratory volume changes comprises:
simulating (310) a muscle activation pattern of the subject based on the subject-specific shape model and biomechanical model;
predicting (312) thorax motion of the subject based on the simulation results;
and
calculating (314) a respiratory volume change based on the predicted thorax motion.

6. The method according to claim 5, wherein predicting (312) thorax motion comprises at least one of:
simulating (320) muscle contraction patterns for a plurality of muscle activation patterns and activation strengths; and
simulating (322) a change in thorax geometry for the muscle contraction patterns.

7. The method according to claim 6, wherein simulating a change in thorax geometry comprises at least one of:
estimating (330) the anatomical cross-sectional area, ACSA, of respiratory muscles from a medical image of the subject's respiratory system;
approximating (332) the direction of muscle fibers based on the subject-specific shape model; and
calculating (334) the physiological cross-sectional area, PCSA, of respiratory muscles.

8. The method according to claim 5, 6 or 7, wherein predicting (140) respiratory volume changes further comprises at least one of:
analyzing one or more medical images from breathing states to identify a muscle activation pattern;
estimating an unknown parameter value based on parameters available from population studies; and
estimating an unknown parameter value by creating a subject-specific computational fluid dynamics, CFD, based flow simulation.

9. The method according to any of claims 1 to 8, wherein an indication of respiratory muscle capability is generated using at least one of:
the quotient of volume change and muscle activation strength;
the quotient of volume change and relative muscle contraction length; and
the pressure change induced by a muscle activation pattern in equilibrium.

10. The method according to any of claims 1 to 9, wherein the medical image comprises:
a computed tomography, CT, scan; or
a magnetic resonance imaging, MRI, scan.

11. The method according to any of claims 1 to 10, wherein the biomechanical model comprises a model created by finite element method, FEM.

12. A computer program product comprising computer program code means which, when executed on a computing device having a processing system, cause the processing system to perform all of the steps of the method according to any of claims 1 to 11.

13. A system for indicating a capability of respiratory muscles of a subject comprising:
an adaption unit (410) configured to adapt a shape model of the respiratory system to a medical image of the subject's respiratory system to obtain a subject-specific shape model;
an interface unit (420) configured to obtain a biomechanical model of the subject's thorax;
a prediction unit (430) configured to predict respiratory volume change of the subject due to muscle contraction based on the subject-specific shape model and biomechanical model; and
a generational (440) unit configured to generate an indication of respiratory muscle capability based on prediction results.

14. The system of claim 13, wherein the adaption unit (410) comprises:
a segmentation component (412) configured to segment the medical image into a plurality of image segments; and
an adaptation component (414) configured to adapt the shape model of the respiratory system to each of the image segments.

15. The system of claim 13 or 14, wherein the prediction unit (430) is configured to:
simulate a muscle activation pattern of the subject based on the subject-specific shape model and biomechanical model;
predict thorax motion of the subject based on the simulation results; and
calculate a respiratory volume change based on the predicted thorax motion.
